# FASCICULE DE BREVET EUROPEEN

(11) **EP 2 900 124 B1**
(45) Date de publication et mention de la délivrance du brevet: **28.07.2021**
(21) Numéro de dépôt: 13779308.9
(22) Date de dépôt: 25.09.2013
(51) Int. Cl.: A61B 5/00, G06T 7/00, G06T 7/49

(54) **MÉTHODE DE CARACTÉRISATION DES VOLUMES DE LA PEAU**
VERFAHREN ZUR KENNZEICHNUNG VON VOLUMINA DER HAUT
METHOD FOR CHARACTERISING VOLUMES OF SKIN

(30) Priorité: 26.09.2012 FR 1259046
(43) Date de publication de la demande: 05.08.2015
(73) Titulaire: LvmH Recherche, 45800 Saint-Jean De Braye (FR)
(72) Inventeur: STEPHAN, Sandrine, F-45190 Beaugency (FR); KORICHI, Rodolphe, F-45650 Saint Jean Le Blanc (FR)
(74) Mandataire: Cabinet Beau de Loménie
(86) Numéro de dépôt international: PCT/FR2013/052263
(87) Numéro de publication internationale: WO 2014/049271

(56) Documents cités:
- WO-A1-2012/001289
- JP-A- 2008 093 048
- KR-A- 20070 032 493
- US-A1- 2011 040 192

## Description

La présente invention a pour objet une méthode de caractérisation de la répartition des volumes de la surface de la peau d'un individu, trouvant plus particulièrement application dans l'évaluation de l'effet de produits cosmétiques raffermissants, restructurants et tenseurs.

La perception visuelle du visage d'une personne et l'âge que l'on peut lui associer dépend en grande partie de la qualité de la répartition des volumes de la peau du visage. Aussi, la perception de la jeunesse d'un visage est fortement impactée par la modification de ses volumes laquelle peut être provoquée par l'apparition de rides ou par le relâchement de la peau consécutif au vieillissement.

Les inventeurs ont développé une nouvelle méthode de caractérisation instrumentale permettant de cartographier au moins une partie de la surface corporelle d'un individu (corps et/ ou visage), et de mesurer ses variations morphologiques. Cette méthode permet de comparer l'évolution de la répartition des volumes de la surface corporelle chez un même individu au cours du temps, ou de comparer la répartition de ces volumes d'un individu à l'autre.

### Art antérieur

La littérature a montré de manière abondante l'intérêt de la technique de Moiré (triangulation optique par décalage de phase) pour cartographier des surfaces et restituer des courbes sur de nombreux supports avec quelques applications au niveau du visage. La technique de Moiré consiste à projeter sur une surface l'ombre de la distribution périodique de formes géométriques telles des franges, des points ou un quadrillage. Les lignes sont produites par la projection de l'ombre des formes géométriques générées par une source de lumière sur la surface dont on souhaite étudier le relief.

Il existe d'autre part différents procédés permettant la détermination de formes tridimensionnelles d'une surface, et notamment celle d'un visage. La plus utilisée est celle de la technique de Moiré associée à la projection de franges.

Enfin, il a été récemment proposé de mesurer l'élasticité de la peau en utilisant une image de Moiré dans la demande KR 2007-32493. La méthode consiste à photographier deux images de Moiré, et l'index d'élasticité peut être défini de deux façons différentes. Selon une première variante, l'image de Moiré correspond à l'ombre de deux cercles projetée sur la surface de la joue gauche du visage. On mesure sur cette première photographie l'écart maximal et l'écart minimal entre les deux cercles, et l'indice d'élasticité correspond au rapport entre l'écart minimal et l'écart maximal entre les deux courbes. Selon une deuxième variante, l'image est la projection d'une ligne horizontale partant de la commissure des lèvres du côté gauche du visage. On mesure l'angle que fait la courbe projetée avec la ligne tangente au contour de lèvres passant à la commissure de lèvres, et l'indice d'élasticité correspond dans ce cas à la valeur de cet angle.

Si ces méthodes permettent de quantifier de manière précise les variations morphologiques comme l'ovale d'un visage ou encore le relâchement des volumes au cours du temps, l'appareil de projection de frange est un appareil volumineux et non portable nécessitant une table de repositionnement pour installer le sujet et deux caméras.

De plus, la restitution faciale des courbes de niveaux en deux dimensions par cette méthode ne permet pas d'atteindre un niveau de résolution satisfaisant. Aucune de ces méthodes n'est assez précise pour visualiser et quantifier les variations morphologiques d'un visage en trois dimensions, plus particulièrement dans la démonstration d'effets cosmétiques.

La méthode décrite dans la demande EP 1 298 562 repose sur la superposition de deux images 3D afin notamment de mesurer l'amincissement de l'ovale du visage ou l'augmentation de la surface des yeux suite à l'application d'un produit tenseur. La méthode de l'invention au contraire comprend le calcul d'un paramètre caractérisant la surface même du visage, et permet de s'affranchir des difficultés liées à la superposition de deux images prises à deux moments différents. La méthode de l'invention permet encore d'effectuer des comparaisons par rapport à un panel de référence, tandis que la méthode de l'art antérieur permet seulement de mesurer l'évolution de l'état de la peau pour un même individu.

Il a été décrit dans la demande US 2011/7040192 une méthode permettant l'étude de la forme, de la couleur et de la topographie de mélanomes. Les caractéristiques topographiques calculées dans de document sont la distance entre le sommet et la hauteur moyenne du mélanome, la distance entre le sommet et l'axe de symétrie principal du mélanome, la distance entre le sommet et le barycentre, et l'aire du mélanome qui se trouve sous un certain seuil de manière à en caractériser la concavité ou la convexité.

Le document WO 2010/001289 divulgue une méthode de détection des des pores et des squames de la peau, par exploitation des lignes de niveaux. Les sommets de la surface sont détectés afin de vérifier si les isolignes qui l'entourent ont un espacement régulier et significatif.

Enfin, la méthode décrite dans la demande JP 2008/093048 exploite des contrastes lumineux et des niveaux de gris pour établir des paramètres morphologiques et évaluer l'élasticité de la peau.

### Description de l'invention

La présente invention fournit une méthode de caractérisation de la répartition ou de la migration des volumes à la surface du corps d'un individu, lesquels volumes sont caractérisés et quantifiés de manière précise, reproductible et significative, en calculant et en comparant certains paramètres caractéristiques du relief de ladite surface. Cette méthode in vivo sans contact permet avantageusement de proposer des paramètres qui se corrèlent de façon significative avec la perception visuelle qu'un expert ou une utilisatrice peut avoir de la surface de la peau.

Selon un de ses aspects, l'invention a donc pour objet une méthode de caractérisation de la répartition ou de la migration des volumes à la surface de la peau d'un individu, ladite méthode comprenant les étapes suivantes:
i) la détermination d'une valeur de référence à partir de l'individu ou d'au moins une autre personne, par
   - acquisition d'une image en trois dimensions sous forme de voxels, représentative de la surface d'au moins une partie du corps dudit individu ou de ladite personne,
   - sélection d'au moins une ligne de ladite image sous la forme d'une surface en trois dimensions, éventuellement assortie de la représentation graphique de cette ligne,
   - calcul d'au moins un paramètre corrélé à ladite ligne, pour obtenir une première valeur appelée valeur de référence,
ii) la détermination d'une deuxième valeur dudit paramètre en appliquant l'étape i) à l'individu, de préférence à un temps différent,
iii) une étape d'estimation de la répartition ou de la migration des volumes à la surface de la peau de l'individu qui consiste à comparer la deuxième valeur à la valeur de référence,
caractérisée en ce que l'acquisition de l'image en trois dimensions est réalisée par stéréoscopie passive et que la surface est en trois dimensions,
caractérisée en ce que l'on sélectionne les lignes d'un maillage polyédrique de l'image, et
caractérisée en ce que le paramètre est la moyenne des directions des gradients des vertex du maillage polyédrique ou la largeur de la distribution des directions des gradients des vertex du maillage polyédrique.

### Prise d'image

Pour étudier la surface du corps, on réalise une image en trois dimensions de la surface corporelle par une saisie dite par « vision passive», par stéréoscopie passive.

La stéréoscopie présente l'avantage de mimer la vision humaine, permettant donc la visualisation de la surface du corps en 3D ainsi que la reconstruction de sa surface en 3D. Le principe de la stéréoscopie passive consiste à reconstruire, via des conditions d'angle de prise et de distance de la zone photographiée standardisées, une scène tridimensionnelle à partir de deux images bidimensionnelles de la scène observée.

Le matériel utilisé est généralement un appareil photographique doté d'un système d'auto-étalonnage ou de calibration absolue. L'auto-calibration permet de restituer la surface avec moins de données que le système de calibration absolue.

Les images sont réalisées de préférence à l'aide d'un appareil photographique capable de réaliser simultanément deux images en deux dimensions en une seule prise de vue, et de construire une image en trois dimensions à l'aide d'un algorithme de reconstruction informatique. L'image est sous forme de voxels.

La surface du corps de l'individu peut être photographiée quelle que soit la position du corps de l'individu, par exemple lorsque l'individu est en position assise, debout ou allongée.

Lorsque l'on souhaite étudier la variation des volumes du visage d'un individu, on préfère prendre la photographie de l'individu en position assise de face.

De façon avantageuse, on délimite une zone d'intérêt de l'image qui a été acquise, et le calcul est réalisé sur ladite zone d'intérêt.

On acquiert par exemple une image de l'ensemble du visage de l'individu et la zone d'intérêt peut alors être une joue, une paupière ou encore l'ovale du visage. La zone d'intérêt de l'image acquise peut être délimitée en appliquant un modèle de référence, transposable d'un individu à l'autre ou pour un même individu d'une prise de vue à l'autre.

Le modèle de référence de la zone d'intérêt a par exemple une valeur de surface donnée, et il est par exemple délimité par au moins un amer anatomique choisi parmi le sillon nasogénien, la position des yeux, le sommet du nez, le sommet du menton et les coins de la bouche.

La zone d'intérêt est de préférence carrée ou essentiellement circulaire, centrée sur la pommette de la joue gauche ou droite de l'individu, et délimitée par le sillon nasogénien.

De façon avantageuse, il peut être défini un modèle de référence des régions anatomiques d'intérêt et de façon à propager ledit modèle, de manière semi-automatique, à l'individu auquel on applique la méthode de l'invention.

Le modèle de référence peut être constitué par une image d'un sujet de référence sur laquelle l'utilisateur de la méthode peut repérer un certain nombre d'amers anatomiques, par exemple la position des yeux, du sommet du nez, des coins de la bouche et du menton.

La mise en correspondance du modèle ainsi défini avec l'image de l'individu peut être réalisée au moyen de la définition de ces mêmes points dans ladite image. La zone d'intérêt peut être définie sur le sujet de référence au moyen de courbes splines qui deviennent disponibles sur l'individu.

### Détermination, affichage et analyse des lignes

Pour fournir une idée du relief, la surface est de préférence représentée par un ensemble de lignes de niveau (ou courbes de niveau, ou encore isolignes), chacune desdites lignes de niveaux étant constituée de l'ensemble des points de même altitude par rapport à un plan de référence que l'utilisateur de la méthode de l'invention peut définir manuellement. On choisit de préférence comme plan de référence le plan perpendiculaire à l'axe de prise de vue passant par le sommet du nez de l'individu.

La forme et l'orientation de ces isolignes permettent de caractériser la morphologie mais également les variations de la surface comme par exemple, celles dues aux migrations des volumes. Des migrations de volume sont souvent consécutives au relâchement de la peau dû au vieillissement : une diminution de la fermeté de la peau la rend plus dépendante de son propre poids, et l'on observe ainsi fréquemment une ptose de la peau du visage par exemple.

La direction des isolignes et leurs courbures permettent aussi de visualiser la déformation morphologique de la surface de la peau, et notamment d'un visage. Par exemple, entre un visage âgé et jeune, on observe des différences dans les valeurs des courbures et la forme des courbures. Les isolignes d'un visage âgé sont plus « fermées » que les isolignes d'un visage jeune qui sont plus régulières et plus « ouvertes ».

Selon un mode de réalisation de l'invention, on sélectionne au moins trois lignes de l'image représentant la surface dont au moins une isoligne, une ligne de crête et une ligne de creux. On sélectionne par exemple un ensemble d'isolignes dont les altitudes sont espacées de 0,1 à 2 mm, de préférence de 1 mm environ.

Le paramètre correspond avantageusement au nombre de points de coin de ladite au moins isoligne, les points de coin correspondant aux points d'intersection de l'isoligne avec la ligne de crête et la ligne de creux. Les points de coin correspondent à des extrêmes de la courbure (minimum ou maximum) le long de l'isoligne.

Le paramètre peut également correspondre au maximum ou au minimum des courbures qui sont mesurées aux points de coin de l'isoligne. On peut introduire dans le calcul des points de coin un paramètre de lissage ainsi qu'un paramètre de seuil de la courbure afin d'éviter la détection de très petites ondulations.

Le paramètre peut encore correspondre à l'amplitude des courbures extrêmes de l'isoligne. Ce paramètre permet de quantifier l'amplitude de la courbure de l'isoligne lorsqu'elle est située sur une bosse (courbure max ou crest curvature) ou dans un creux (courbure min).

### Détermination des directions des volumes

La surface du corps de l'individu est représentée par les lignes d'un maillage polyédrique appartenant à l'image.

On calcule les gradients de chacun des vertex de ce maillage polyhédrique, et le paramètre est la moyenne des directions des gradients des vertex du maillage polyédrique, ou la largeur de la distribution des directions des gradients des vertex du maillage polyédrique.

La mesure des directions principales des volumes illustrés par les isolignes peut être effectuée en calculant le vecteur gradient pour chaque voxel de l'image ou pour chaque vertex du maillage polyhédrique. Le vecteur gradient a une composante x (projeté du vecteur sur l'axe horizontale) et un composante y (projeté du vecteur sur l'axe vertical correspondant à l'axe de prise de vue). Si la variation des directions du volume est importante, la norme du vecteur est importante. Si le changement de direction est plus important par rapport à l'axe horizontal, la composante en x devient plus importante que la composante en y. Enfin, si la variation de direction du volume est plus importante par rapport à l'axe vertical, la composante en y devient plus importante que la composante en x.

La direction des gradients peut être mesurée à partir de l'image saisie et par rapport à un axe de référence, comme par exemple, un angle de 45°.

L'angle θ (angle du vecteur gradient par rapport à l'horizontal encore appelé direction du gradient) est notamment représentatif de la prédominance d'une des composantes (x ou y): si θ=45° alors x=y, si θ > 45 alors y > x et si θ <45 alors x >y.

Plus la valeur algébrique de l'angle moyen des gradients est élevée, plus la peau est ptosée.

La répartition des directions du gradient correspond à l'étalement de l'histogramme des directions du gradient. Plus il y a de directions de gradient différentes sur une zone, plus l'histogramme a un étalement important et plus la zone étudiée a une surface irrégulière.

Tous les paramètres qui viennent d'être décrits peuvent être calculés selon les formules données dans la thèse intitulée « Mise en correspondance automatique d'images médicales tri-dimensionnelles », Jean-Philippe Thirion, Habilitation à Diriger des Recherches, 5 juin 1997, Université de Nice, Sophia-Antipolis.

La valeur de référence du paramètre peut être déterminée à partir de la valeur du paramètre calculée sur au moins deux personnes différentes appartenant à un groupe de référence. Le groupe de référence peut être par exemple défini comme étant une classe d'âge d'individus de même sexe. La valeur de référence peut être la moyenne des au moins deux valeurs obtenues dans le groupe de référence.

On choisira de préférence un groupe de référence dont le sexe et la classe d'âge correspond à celle de l'individu. On pourra également choisir un groupe de référence dont les personnes ont des amers anatomiques dans une configuration proche de celle de l'individu.

La méthode de l'invention peut comprendre la détermination d'au moins deux valeurs de paramètres. La première mesure et la deuxième mesure ont lieu à un temps différent au sens où, entre ces deux mesures, s'écoule un laps de temps plus ou moins long pendant lequel l'état de l'individu se trouve modifié par rapport à un état dit « de référence » pour la mise en oeuvre de la méthode de l'invention. Ainsi ce laps de temps peut être de quelques minutes mais peut aller jusqu'à plusieurs années, selon l'état retenu.

Ainsi selon une alternative de la méthode de l'invention, la valeur de référence du paramètre est calculée selon la méthode pour un premier état de l'individu, et le paramètre est ensuite est calculé pour au moins un autre état du même individu. L'état de l'individu peut être son âge ou la position de son corps lors de l'acquisition de l'image.

L'état de référence peut être antérieur à l'application d'un traitement cosmétique et que l'acquisition de l'image peut être postérieure à l'application d'un traitement cosmétique.

La méthode de l'invention peut, dans cette alternative, être mise en œuvre pour évaluer la ptose du visage d'un individu due au vieillissement au cours du temps.

L'invention concerne également une méthode d'évaluation de l'efficacité d'un agent cosmétique ou d'une composition cosmétique, en particulier pour améliorer l'état de fermeté et de tension de la peau, pour lutter contre le relâchement de la peau, ou pour lutter contre la perte d'élasticité de la peau d'au moins un individu en ayant besoin, caractérisée en ce que l'on met en œuvre la méthode de caractérisation décrite précédemment
- en appliquant ledit agent cosmétique ou ladite composition sur la peau de l'individu entre les étapes i) et ii), et
- en concluant à l'efficacité ou à l'absence d'efficacité dudit agent cosmétique après l'étape de comparaison iii).

Le produit cosmétique peut être notamment un produit tenseur, raffermissant ou anti-âge. Ladite méthode de d'évaluation de l'efficacité peut être mise en œuvre pour évaluer les effets de l'application d'un produit cosmétique tenseur, raffermissant ou anti-âge sur la modification de la répartition des volumes du visage d'un individu, en particulier sur la forme de l'ovale du visage.

Selon encore un autre de ses aspects, l'invention a pour objet une méthode d'évaluation de l'évolution du niveau de fermeté ou de relâchement de la peau chez un même individu, ladite méthode d'évaluation comprenant la mise en œuvre de la méthode de caractérisation décrite précédemment dans laquelle la valeur de référence est déterminée pour un premier état de l'individu.

L'invention et décrite plus en détail en référence aux figures et exemples suivants.
La figure 1 reproduit un maillage de vertex carré représentant la surface du visage d'un individu.
La Figure 2 représente les isolignes, les lignes de crêtes et les points de coins de la surface du visage d'un individu.

### Exemple :

Le visage de 40 femmes âgées de 21 à 86 ans n'ayant subi aucune opération de chirurgie esthétique a été photographié dans des conditions de lumière, de distance et d'angle standardisées.

Pour la prise de vue en stéréoscopie passive on utilise deux appareils photos photographiques identiques à visée optique équipé d'un capteur CMOS 18 millions de pixels (5184 x 3456 pixel) et d'un objectif de focale fixe 60 mm. Les deux appareils sont positionnés à même distance de l'individu photographié, et à 30° de part et d'autre de l'axe médian perpendiculairement au plan de la zone à photographier.

La zone d'intérêt est un rectangle de 5 cm sur 4 cm positionné en diagonale sur la joue.

La photo est prise bouche fermée, cheveux attachés pour dégager le visage. La prise de vue est déclenchée simultanément pour les deux appareils de façon à obtenir deux photographies à un angle de 60° l'une de l'autre.

Deux groupes ont été constitués : un premier groupe de 20 femmes (âge moyen = 32 ans +/-10 ans) et un second groupe de 20 femmes (âge moyen = 59 ans +/-20 ans).

L'appareil était doté d'un système de calibration absolu et paramétré pour un champ de vision de 20x20cm.
Paramètres de l'appareil pour la prise de vue:
▪ 1/200 - shutter speed.
▪ F00 - aperture.
▪ M - indicates manual setting mode.
▪ Flash exposure depending on ambient light.
▪ Iso 100 - camera light sensitivity mode.
▪ "Flash" white balance.
▪ Partial metering mode.
▪ One shot.
▪ L - indicates image storage quality.
▪ Central manual auto focus point selected.

L'axe et la distance de prise de vue sont définis en pointant la base des narines de chaque femme avec deux rayons lumineux émis par l'appareil. Une fois les deux rayons confondus, l'appareil est déplacé pour que les deux rayons atteignent le sommet du nez et la photo est prise.

Une image en trois dimensions a été reconstituée de façon classique à partir des deux images prises par l'appareil. Cette image peut être visualisée avec le logiciel Cortona VRML. On a choisi une zone d'intérêt sur la joue droite ou gauche des images en deux dimensions proche du sillon nasogénien de la première femme du groupe. Cette zone est un rectangle de 5cm sur 4 cm, positionné en diagonale sur la joue comme indiqué sur le doc de l'étude. Cette zone d'intérêt a ensuite été propagée sur toutes les autres images.

Les isolignes distantes de 1 mm ont été calculées par rapport au plan passant par le sommet du nez et perpendiculaire à l'axe de prise de vue.

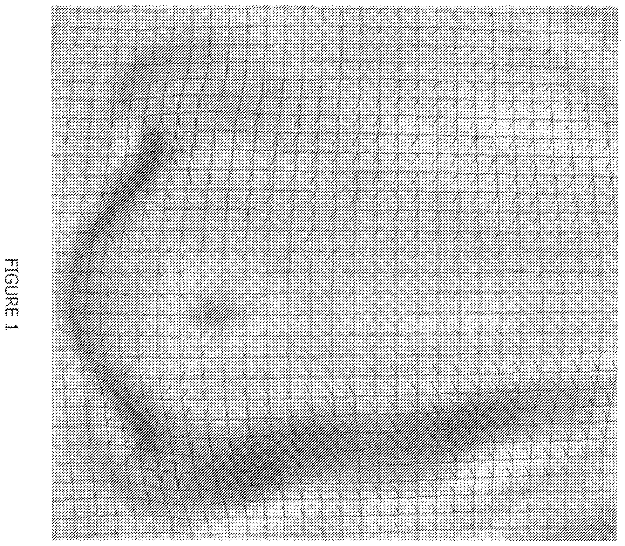

Un maillage de vertex carré de côté égal à 1 mm a également été calculé et représenté (voir la figure 1).

La direction des courbures a été calculée à partir des vecteurs gradients sur le maillage de vertex et une direction de référence précisée par l'utilisateur. La valeur a été exprimée en degrés allant de -180 à +180.

Les valeurs des courbures ont été calculées à partir de l'angle entre le gradient de surface et le plan horizontal (plan correspondant au plan de face). La valeur a été exprimée en degrés allant de 0 à 90. 0 correspond à une courbure nulle et 90 à une surface verticale.

Chaque calcul a été réalisé en utilisant le résultat sigma=2 pour ne pas introduire trop de lissage sur le maillage d'origine et pour désactiver l'option de « fix planarity ». Tous les angles s'expriment en degrés.

Sur la partie droite du visage, la direction moyenne des courbures est égale à -21,9° ce qui signifie que cette direction moyenne est légèrement inférieur à la direction de référence 45°. Sur la partie gauche la valeur symétrique est égale à 24,1° par rapport à la direction de référence 135°.

Les résultats entre les parties droite et gauche sont assez différents car l'axe Z ne correspond pas exactement à l'axe du visage. En conséquence, les valeurs de gradient ainsi que les isolignes ne sont pas symétriques.

La fonction de la courbure d'isoligne a été également examinée sur la surface 3D. La dérivée de cette fonction y est également disponible et est utilisée afin de déterminer les extrêmes de la fonction de courbure. En effet, les positions dans lesquelles la fonction a une valeur nulle définissent les courbures qui correspondent à l'incurvation des courbes aux extrémités, aux crêtes et aux creux de la surface.

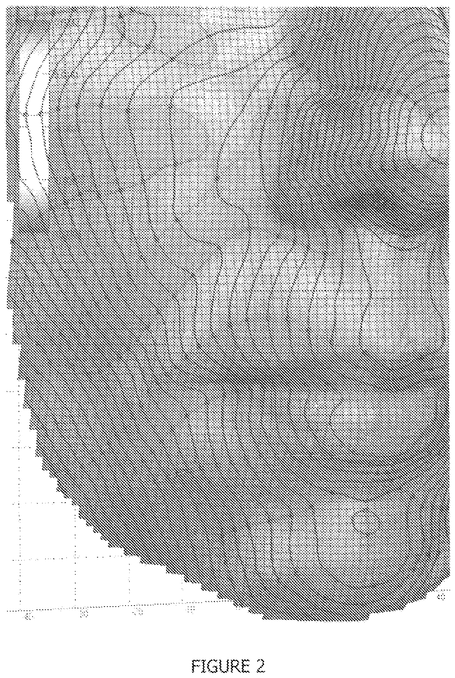

Le nombre de points de coin correspond au nombre des intersections entre les isolignes et l'incurvation des courbes aux extrémités. « Trough curvature » et « Crest curvature » correspondent à la valeur d'incurvation aux extrémités sur ces points. Pour ce calcul, l'espacement d'isoligne était à 1 mm avec une valeur sigma=3 (voir la figure 2).

**TABLEAU 1**

| | Visage jeune (n=20, moyenne 32 ans) | Visage âgé (n=20, moyenne 59 ans) | *Significativité* |
|---|---|---|---|
| Direction moyenne du gradient (par rapport à l'angle de référence 45°, joue droite) | -26.2° | -17.6° | S (p=0,02) |
| Variation moyenne du gradient (par rapport à l'angle de référence 45°, joue droite) | 21.1° | 29.5° | S (p<0,01) |
| «Crest curvature» moyenne | 0.17 | 0.35 | S (p=0,02) |
| «Tough curvature» moyenne | 0.16 | 0.24 | S (p=0,03) |
| Nombre de points de coin | 36 | 34.7 | S (p<0,03) |

Globalement, les résultats montrent que l'étude des gradients et des courbures permet de distinguer le groupe des femmes jeunes et le groupe des femmes âgées. Ces paramètres sont donc bien représentatifs des phénomènes physiologiques du vieillissement, plus particulièrement de la ptose d'un visage.

La méthode de l'invention permet des mesures objectives du vieillissement cutané.

Les joues des personnes âgées donnent l'impression de s'affaisser ce qui se traduit ici par une direction de pente maximale inférieure. On observe également que plus la peau est lâche, plus les directions de la courbure maximale sont étalées. La courbure des isolignes s'accroît également avec l'âge.

## Revendications

1. Méthode de caractérisation de la répartition ou de la migration des volumes à la surface de la peau d'un individu, ladite méthode comprenant les étapes suivantes:
i) la détermination d'une valeur de référence à partir de l'individu ou d'au moins une autre personne, par
- acquisition d'une image en trois dimensions sous forme de voxels représentative de la surface d'au moins une partie du corps dudit individu ou de ladite personne,
- sélection d'au moins une ligne de ladite image sous la forme d'une surface en trois dimensions, éventuellement assortie de la représentation graphique de cette ligne,
- calcul d'au moins un paramètre corrélé à ladite ligne, pour obtenir une première valeur appelée valeur de référence,
ii) la détermination d'une deuxième valeur dudit paramètre en appliquant l'étape i) à l'individu,
iii) une étape d'estimation de la répartition ou de la migration des volumes à la surface de la peau de l'individu qui consiste à comparer la deuxième valeur à la valeur de référence,
**caractérisée en ce que** l'acquisition de l'image en trois dimensions est réalisée par stéréoscopie passive et que la surface est en trois dimensions,
**caractérisée en ce que** l'on sélectionne les lignes d'un maillage polyédrique de l'image, et
**caractérisée en ce que** le paramètre est la moyenne des directions des gradients des vertex du maillage polyédrique ou la largeur de la distribution des directions des gradients des vertex du maillage polyédrique.

2. Méthode de caractérisation selon la revendication 1, **caractérisée en ce que** l'on sélectionne au moins trois lignes dont une isoligne, une ligne de crête et une ligne de creux.

3. Méthode de caractérisation selon la revendication 2, **caractérisée en ce que** le paramètre est le nombre de points de coin de l'isoligne.

4. Méthode de caractérisation selon la revendication 2, **caractérisée en ce que** le paramètre correspond au maximum ou au minimum des courbures qui sont mesurées aux points de coin de l'isoligne.

5. Méthode de caractérisation selon l'une des revendications précédentes, **caractérisée en ce que** l'on délimite une zone d'intérêt de l'image qui a été acquise, et que le calcul est réalisé sur ladite zone d'intérêt.

6. Méthode de caractérisation selon l'une des revendications 1 à 5, **caractérisée en ce que** l'étape i) est mise en œuvre sur au moins deux personnes différentes appartenant à la même classe d'âge, et que la valeur de référence est la moyenne des au moins deux valeurs obtenues.

7. Méthode d'évaluation de l'efficacité d'un agent cosmétique ou d'une composition cosmétique pour améliorer l'état de fermeté de la peau, pour lutter contre le relâchement de la peau, ou pour lutter contre la perte d'élasticité de la peau d'au moins un individu en ayant besoin, **caractérisée en ce que** l'on met en œuvre la méthode de caractérisation selon l'une des revendications 1 à 6,
- en appliquant ledit agent cosmétique ou ladite composition sur la peau de l'individu entre les étapes i) et ii), et
- en concluant à l'efficacité ou à l'absence d'efficacité dudit agent cosmétique après l'étape de comparaison iii).

8. Méthode d'évaluation de l'évolution du niveau de fermeté ou de relâchement de la peau chez un même individu, ladite méthode d'évaluation comprenant la mise en œuvre de la méthode de caractérisation selon l'une des revendications 1 à 6 dans laquelle la valeur de référence est déterminée pour un premier état de l'individu.

## Patentansprüche

1. Verfahren zur Kennzeichnung der Verteilung oder der Migration von Volumina auf der Hautoberfläche eines Individuums, wobei das Verfahren die folgenden Schritte umfasst:
i) die Bestimmung eines Bezugswerts ausgehend von dem Individuum oder mindestens einer anderen Person, durch
- Aufnahme eines dreidimensionalen Bildes in Form von Voxeln, welche die Oberfläche von mindestens einem Teil des Körpers des Individuums oder der Person darstellen,
- Auswahl von mindestens einer Linie des Bildes in Form einer dreidimensionalen Oberfläche, eventuell versehen mit der grafischen Darstellung dieser Linie,
- Berechnung mindestens eines mit der Linie korrelierten Parameters, um einen ersten Wert zu erhalten, der als Bezugswert bezeichnet wird,
ii) die Bestimmung eines zweiten Werts des Parameters unter Anwendung des Schritts i) auf das Individuum,
iii) einen Schritt des Schätzens der Verteilung oder der Migration von Volumina auf der Hautoberfläche des Individuums, der in dem Vergleichen des zweiten Werts mit dem Bezugswert besteht,
**dadurch gekennzeichnet, dass** die Aufnahme des dreidimensionalen Bildes durch eine passive Stereoskopie umgesetzt wird und dass die Oberfläche dreidimensional ist,
**dadurch gekennzeichnet, dass** die Linien einer polyedrischen Vernetzung des Bildes ausgewählt werden, und
**dadurch gekennzeichnet, dass** der Parameter der Mittelwert der Richtungen der Gradienten der Scheitelpunkte der polyedrischen Vernetzung oder der Breite der Richtungsverteilung der Gradienten der Scheitelpunkte der polyedrischen Vernetzung ist.

2. Verfahren zur Kennzeichnung nach Anspruch 1, **dadurch gekennzeichnet, dass** mindestens drei Linien ausgewählt werden, darunter eine Isolinie, eine Kammlinie und eine Troglinie.

3. Verfahren zur Kennzeichnung nach Anspruch 2, **dadurch gekennzeichnet, dass** der Parameter die Anzahl der Eckpunkte der Isolinie ist.

4. Verfahren zur Kennzeichnung nach Anspruch 2, **dadurch gekennzeichnet, dass** der Parameter höchstens oder mindestens den Krümmungen entspricht, die an den Eckpunkten der Isolinie gemessen werden.

5. Verfahren zur Kennzeichnung nach einem der vorhergehenden Ansprüche, **dadurch gekennzeichnet, dass** ein interessierender Bereich des Bildes, das aufgenommen wurde, eingegrenzt wird, und dass die Berechnung an dem interessierenden Bereich umgesetzt wird.

6. Verfahren zur Kennzeichnung nach einem der Ansprüche 1 bis 5, **dadurch gekennzeichnet, dass** der Schritt i) für mindestens zwei unterschiedliche Personen in die Tat umgesetzt wird, die zu der gleichen Altersgruppe gehören, und dass der Bezugswert der Mittelwert der mindestens zwei erhaltenen Werte ist.

7. Verfahren zur Bewertung der Wirksamkeit eines kosmetischen Mittels oder einer kosmetischen Zusammensetzung, um den Zustand der Hautfestigkeit zu verbessern, um gegen die Erschlaffung der Haut zu kämpfen oder um gegen den Elastizitätsverlust der Haut von mindestens einem Individuum zu kämpfen, das dessen bedarf, **dadurch gekennzeichnet, dass** das Verfahren zur Kennzeichnung nach einem der Ansprüche 1 bis 6 in die Tat umgesetzt wird,
- indem das kosmetische Mittel oder die Zusammensetzung auf der Haut des Individuums zwischen den Schritten i) und ii) angewendet wird, und
- indem nach dem Vergleichsschritt iii) Schlussfolgerungen hinsichtlich der Wirksamkeit oder des Fehlens der Wirksamkeit des kosmetischen Mittels gezogen werden.

8. Verfahren zur Bewertung der Entwicklung des Niveaus der Festigkeit oder der Erschlaffung der Haut bei demselben Individuum, wobei das Verfahren zur Bewertung das Umsetzen des Verfahrens zur Kennzeichnung nach einem der Ansprüche 1 bis 6 in die Tat umfasst, wobei der Bezugswert für einen ersten Zustand des Individuums bestimmt wird.

## Claims

1. A method for characterizing the distribution or the migration of volumes on the surface of the skin of an individual, said method comprising the following steps:
i) determining a reference value from the individual or from at least one other person, by
- acquiring a three-dimensional image in voxel form that represents the surface of at least one part of the body of said individual or of said person,
- selecting at least one line of said image in the form of a three-dimensional surface, and eventually making the graphic representation of this line,
- computing at least one parameter correlated with said line, and obtaining a first value, called reference value,
ii) determining a second value of said parameter by applying step i) to the individual,
iii) a step of estimating the distribution or the migration of the volumes on the surface of the skin of the individual, said step consisting in comparing the second value to the reference value,
**characterized in that** acquiring a three-dimensional image is realized with passive stereoscopy, and **in that** the surface is a three-dimensional surface,
**characterized in that** lines of a polyhedral meshing of the image are selected, and
**characterized in that** the parameter is the mean of the directions of the gradients of the vertices of the polyhedral meshing, or the width of the distribution of the directions of the gradients of the vertices of the polyhedral meshing.

2. The characterization method as claimed in claim 1, **characterized in that** at least three lines are selected: namely an isoline, a peak line and a trough line.

3. The characterization method as claimed in claim 2, **characterized in that** the parameter is the number of corner points of the isoline.

4. The characterization method as claimed in claim 2, **characterized in that** the parameter corresponds to the maximum or to the minimum of the curvatures which are measured at the corner points of the isoline.

5. The characterization method as claimed in one of the preceding claims, **characterized in that** an area of interest is delimited from the image which has been acquired, and **in that** the computation is performed on said area of interest.

6. The characterization method as claimed in one of claims 1 to 5, **characterized in that** the step i) is implemented on at least two different persons belonging to the same age category, and that the reference value is the mean of the at least two values obtained.

7. A method for evaluating a cosmetic agent or a cosmetic composition regarding its effectiveness to improve the state of firmness of the skin, to counter the relaxation of the skin, or to counter the loss of elasticity of the skin of at least one individual in need thereof, **characterized in that** the characterization method as claimed in one of claims 1 to 6 is implemented:
- by applying said cosmetic agent or said composition to the skin of the individual between steps i) and ii), and
- by concluding on the effectiveness or the lack of effectiveness of said cosmetic agent or said composition after the comparison step iii).

8. A method for evaluating the trend of the firmness level or of the relaxation level of the skin in one and the same individual, said evaluation method comprising the implementation of the characterization method as claimed in one of claims 1 to 6 in which the reference value is determined for a first state of the individual.
